(19) Europäisches Patentamt
European Patent Office
Office européen des brevets

(11) Publication number: **0 163 405**
**A2**

(12) # EUROPEAN PATENT APPLICATION

(21) Application number: 85302733.2

(51) Int. Cl.[4]: **C 07 H 21/00**

(22) Date of filing: 18.04.85

(30) Priority: 20.04.84 US 602287
20.07.84 US 632919

(43) Date of publication of application:
04.12.85 Bulletin 85/49

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Froehler, Brian Carl
Carlmont Drive
Belmont California 94002(US)

(72) Inventor: Matteucci, Mark Douglas
56 Telegraph Place
San Francisco California 94133(US)

(74) Representative: Armitage, Ian Michael et al,
MEWBURN ELLIS & CO. 2/3 Cursitor Street
London EC4A 1BQ(GB)

(54) **Method of rate enhancement for triester synthesis of oligonucleotides using a catalytic alcohol, and compounds for use therein.**

(57) The rate of the triester method of oligonucleotide synthesis is increased by using a phosphotriester formation catalyst/blocking group diester linked to the 3' phosphate of a nucleotide (or an oligonucleotide), thus forming a nucleotide phosphodiester:

Insert here

wherein Base is a nucleotide base;

wherein Pr is a nucleotide 5' hydroxyl protecting group, for example, monomethoxytrityl or dimethoxytrityl;

wherein R includes a substituted or unsubstituted, aryl, alkyl, cycloalkyl or beta cyanoalkyl blocking group;

wherein CAT is a catalytic moiety, which is, for example, a substituted or unsubstituted, nitrogen heterocycle, tertiary amine or a carboxylic acid.

The use of these groups so linked to form nucleotide phosphodiesters enhances the rate of an internucleotide condensation reaction. Following condensation (which results in the formation of a phosphotriester) the phosphotriester formation catalyst/blocking group can be removed leaving a di-nucleotide, or the condensation reaction can be repeated to synthesize an oligonucleotide.

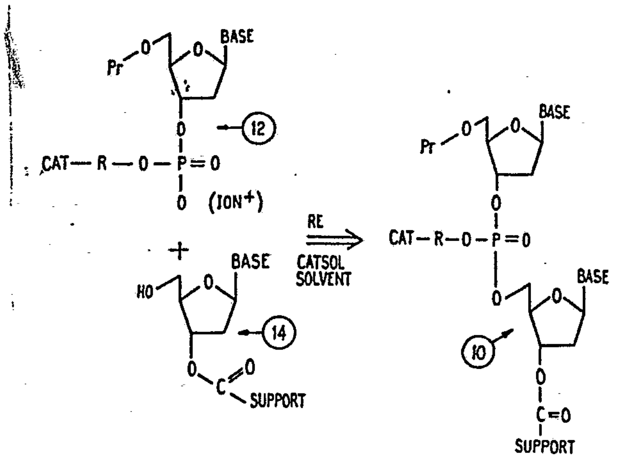


CONDENSATION REACTION - 8

FIG.1

EP 0 163 405 A2

# METHOD OF RATE ENHANCEMENT FOR TRIESTER SYNTHESIS OF OLIGONUCLEOTIDES USING A CATALYTIC ALCOHOL, AND COMPOUNDS FOR USE THEREIN

The invention relates to rate enhancement of phosphotriester formation in the triester method of oligonucleotide synthesis using a 3' phosphate blocking group which also acts as a catalyst.

Synthesis of oligonucleotides, or deoxyoligonucleotides (d-oligonucleotides), essentially entails the formation of a linear polymeric sugar phosphodiester. To accomplish this formation, ester bonds must be sequentially formed by linking the phosphate group of a nucleotide or a deoxynucleotide (d-nucleotide), with the 5' hydroxyl group of another nucleotide or nucleoside. In order to form a nucleotide chain, one must ensure that the bond is formed at the appropriate 3' phosphate (which should be at the terminal position of a first nucleotide) and also that the bond is formed with the 5' hydroxyl group of a second nucleotide and not at one of the other reactive sites of its sugar residue. These dual aims are accomplished by selective, sequential protection and deprotection of the desired reaction sites.

In the triester method of d-oligonucleotide synthesis, frequently used as a preferred synthesis method, a triester of phosphoric acid is formed with the establishment of each internucleotide linkage. Typically, the triester is formed by first forming a d-nucleotide phosphodiester involving the 3' phosphate group of the terminal nucleotide and an aryl blocking group. Aryl blocking groups commonly used are ortho or para chlorophenyl derivatives; beta-cyanoalkyl has also been found to be an effective blocking group. The 5' hydroxyl group of this d-nucleotide phosphodiester is then blocked, typically with a monomethyoxytrityl or dimethoxytrityl group, to prevent unwanted linkages from occuring at this site. Heterocyclic amines, such as 5H-tetrazole, N-methyl imidazole or 4-N, N-dimethylamino pyridine, are added as catalysts, and the protected d-nucleotide phosphodiester is condensed with the 5' hydroxyl group of another d-nucleotide to form a d-nucleotide phosphotriester. The aryl blocking group can thereafter be removed leaving a di-d-nucleotide or a d-oligonucleotide. The 5' hydroxyl group can be detritylated to leave a deprotected d-nucleotide or d-oligonucleotide. (See Crockett, The Chemical Synthesis of DNA, Aldrichimica Acta, Vol. 16, No. 3, 1983).

Additionally, it has been found that the rate of inter-nucleotide condensation can be increased if aryl sulfonylchlorides in the presence of the N-Methyl imidazole, all in a solution of organic solvents such as acetonitrile, methylene chloride, pyridine or dioxane, are used as coupling reagents. (See, Efimov, Reverdatto, Chakhmakhcheva, New Effective Method for the Synthesis of Oligonucleotides via Phosphotriester Intermediates, Nucleic Acids Research, Vol. 10, No. 82, 1982).

Among the problems associated with triester method of d-oligonucleotide synthesis is that there is a low rate of reactivity between the d-nucleotides, which makes it difficult and time consuming to form the multiple, sequential linkages between the d-nucleotides necessary for synthesis of complex d-oligonucleotides. With the use of aryl sulfonylchlorides as coupling reagents an additional problem arises because the sulfonylchloride group will react with the hetrocyclic bases of the nucleotides (this reaction is most prevalent at the 0-6 position of the guanine base) and thereby result in a chemical modification of the base and a lower purity of end product, (See Crockett at 52, cited supra). Additionally, the unprotected 5' hydroxyl groups will react with aryl sulfonylchloride and thereby lower the product yield. Finally, the exocyclic nitrogens on the nucleotide bases are nucleophilic and can participate in side reactions unless they are protected.

Thus, what is needed is a process which will allow for more rapid internucleotide linkages thereby making synthesis of oligonucleotides less time consuming and more practical. More rapid inter-nucleotide linkages would further serve to reduce side reactions at the 3' phosphate, side reactions between the sulfonylchloride coupling reagents and the heterocyclic bases, and side reactions between the sulfonylchloride coupling reagents and the unprotected 5' hydroxyl groups. More rapid linkages would also reduce any side reactions involving exocyclic nitrogens, thereby yielding a higher purity of end product.

The invention includes a compound which is a nucleotide having a diester bonded catalytic moiety/blocking group at its 3' phosphate, a method of using the compound in an internucleotide condensation reaction as a phosphotriester formation catalyst, and the phosphotriester which is one of the condensation reaction products. If the compounds of the invention are used as starting materials with the standard techniques and reagents of the triester method of DNA synthesis, the rate of phosphotriester formation can be increased.

The compounds of the invention have the formula:

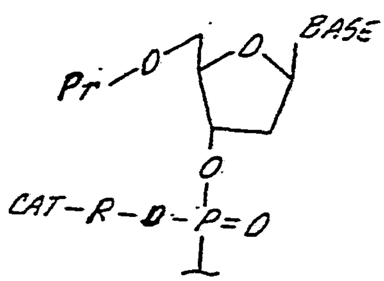

wherein Base is a nucleotide base;

wherein Pr is a nucleotide 5' hydroxyl protecting group, for example, monomethoxytrityl or dimethoxytrityl;

wherein R includes a substituted or unsubstituted, aryl, alkyl, cycloalkyl or beta cyanoalkyl blocking group;

wherein CAT is a catalytic moiety, which is, for example, a substituted or unsubstituted, nitrogen heterocycle, tertiary amine or a carboxylic acid.

The catalytic moieties N-methyl imidazole and tetrazole have been found to be particularly effective for rate enhancement when linked to the ortho position of a phenyl group. The diester bonded blocking groups of the compounds of the invention can be any aryl, alkl, cycloalkyl, or beta cyanoalkyl group. Nitrogen heterocycles and/or tertiary amines other than tetrazole and imidazole, when linked to a diester bonded blocking group, also result in an increased rate of

nucleotide phosphotriester formation. Furthermore, it is believed that carboxylic acids will increase the rate of phosphotriester formation if properly positioned.

Thus, in the preferred embodiments of the invention R-CAT is either 2-(2'-hydroxyphenyl) N-methyl imidazole or 5-(2'-hydroxyphenyl) tetrazole wherein the hydroxyl groups of each of these compounds is replaced by a diester bond to the 3' phosphate of the nucleotide. A significant rate enhancement of an internucleotide condensation reaction also results if R-CAT is any of the following catalytic alcohols, each of which is diester bonded to the 3' phosphate of a nucleotide: 8-hydroxy quinoline; (2-hydroxy ethyl) N,N,dimethyl amine; (3-hydroxy propyl) N,N-dimethyl amine; 2-(hydroxy methyl) pyridine; 2-(2'-hydroxy ethyl) pyridine; 2-(hydroxy methyl) N-methyl imidazole; 2-(2-hydroxy ethyl) N-methyl Imidazole; or 5-(2'-hydroxy ethyl) tetrazole.

R-CAT can be broadly defined as any catalytic alcohol diester bonded to the 3' phosphate of a nucleotide, wherein the catalytic moiety is positioned such that there is an enhanced rate of internucleotide condensation as compared to an internucleotide condensation reaction utilizing a parachlorophenyl 3' phosphate blocking group and, in solution, the catalyst N-methyl imidazole. It is hypothesized that R-CAT functions by enabling neighboring group participation and perhaps, more specifically, by enabling the formation of an active cyclic species with the 3' phosphate, wherein this active cyclic species can undergo nucleophilic attack from a 5' hydroxyl group. This hypothesized active cyclic species is thought to

be a heterocyclic intermediate compound wherein the 3' phosphorous and the diester bonded oxygen are two of the hetrocycle ring atoms, and particular atoms of the catalytic alcohol make up the remainder of the heterocycle ring.

It is believed that the rate enchancement of nucleotide phosphotriester formation results from the correct positioning of the catalytic moiety relative to the 3' phosphate group rather than from the nature of the catalytic moieties and the blocking groups. It is hypothesized that when the blocking group is a phenyl or a phenyl derivative, for example, placing the catalytic moiety at the ortho position thereof allows neighboring group participation and the formation of an active cyclic species.

It is an object of this invention to increase the rate of an internucleotide condensation reaction and thereby make synthesis of complex oligonucleotides more practical and less time consuming. The increased rate of reaction will help reduce side reactions at the reactive sites of the phosphodiester and will also help reduce side reactions between the unprotected 5' hydroxyl groups and the sulfonyl chlorides thereby increasing the purity of the yield of the final oligonucleotide product.

In the drawings:

FIG. 1 is a schematic representation of an internecleotide condensation reaction.

FIG. 2 is a schematic representation of the postulated reaction mechanism using 5-(2'-hydroxyphenyl) tetrazole as the catalytic alcohol in an internucleotide condensation reaction.

FIG. 3 is a schematic representation of the postulated reaction mechanism using 2-(2'-hydroxyphenyl) N-methyl imidazole as the catalytic alcohol in an inter-nucleotide condensation reaction.

FIG. 4 is a schematic representation of the postulated reaction mechanism when using (2-hydroxy ethyl) N,N-dimethyl amine as the catalytic alcohol in an inter-nucleotide condensation reaction.

FIG. 5 shows the relative reactivity rates of inter-nucleotide condensation using two of the preferred diester-bonded catalytic alcohols as compared with another embodiment and a standard blocking group.

FIG. 6 schematically shows the reaction of danzyl alcohol with a thymidine phosphodiester.

FIG. 7 shows the P31 NMR spectra following reaction of the 5-(2'-hydroyphenyl) tetrazole diester of thymidine with various reagents.

FIG. 8 shows the P31 NMR spectra following reaction of the para-chlorophenyl diester of thymidine with various reagents.

FIG. 9 shows an autoradiogram following gel electrophoresis of a thymidine (T-20) standard, as well as an

autoradiogram following a multiple, sequential, series of internucleotide condensation reactions using two of the preferred diester-bonded catalytic alcohols.

Referring to FIG. 1, a condensation reaction 8 is shown, which results in the formation of a phosphotriester 10. In reaction 8 a protected phosphodiester 12, having a catalytic alcohol of the invention diester bonded thereto, is reacted with a compound 14 -- shown in this embodiment of reaction 8 as a nucleoside carboxyl bonded to a support. The groups shown attached at the various positions to protected phosphotriester 10, protected phosphodiester 12, and compound 14 are as follows.
"Pr" represents a known 5' hydroxyl protecting group including, but not limited to, dimethoxytrityl and monomethoxytrityl.
"Base" represents a nucleotide base including Thymine, Uracil and suitably protected Adenine, Guanine and Cytosine.
"R" represents a 3' phosphate blocking group upon which the catalytic moiety can be positioned so as to lead to rate enhancement of the formation of phosphotriester 10; R includes groups such as, aryl, alkyl, cycloalkyl and beta cyanoalkyl.
"CAT" represents a rate-enhancing catalytic moiety which is thought to form an active cyclic species with the 3' phosphate of protected phosphodiester 12 that is capable of reacting with the 5' hydroxyl group of compound 14 (or any other nucleotide,

nucleoside, oligonucleotide or oligonucleoside, or the deoxy species of any of these). CAT includes carboxylic acids, substituted or unsubstituted nitrogen heterocycles, or substituted or unsubstituted tertiary amines.

Generally "R-CAT" represents a diester bonded rate-enchancing catalytic alcohol (which also functions as a blocking group), and includes the following:

8-hydroxy quinoline,

(2-hydroxy ethyl) N,N,dimethyl amine,

(3-hydroxy propyl) N,N-dimethyl amine,

2-(hydroxy methyl) pyridine,

2-(2'-hydroxy ethyl) pyridine,

2-(hydroxy methyl) N-methyl imidazole,

2-(2'-hydroxy ethyl) N-methyl imidazole, or

5-(2'-hydroxy ethyl) tetrazole.

In a preferred embodiment, R-CAT is either 2-(2'-hydroxyphenyl) N-methyl imidazole, or 5-(2'-hydroxyphenyl) tetrazole.

"(Ion)" represents any positive ion, including triethylamine H.

"Support" represents an insoluble support for a nucleotide or nucleoside, including silica gel.

"Re" represents any dehydrating agent, including, but not limited to, an aryl sulfonylchloride, an alkyl sulfonylchloride, mesitylene sulfonylchloride, ortho nitro-benzene sulfonylchloride, toluene sulfonylchloride, an aryl or alkyl sulfonylbromide, a carbodiimide compound or mesitylene nitrotriazole.

"CATSOL" represents a catalyst free in solution which facilitates an inter-nucleotide condensation reaction.

"Solvent" represents an organic solvent capable of buffering an acid formed during the condensation reaction 8, pyridine being one such solvent, and further including any such solvent mixed with another solvent which is inert to the reagents of the reaction, such inert solvents including acetonitrile and methylene chloride.

Description of the Postulated Reaction Mechanisms

Many of the groups shown in FIG. 1 have been described in terms of their function in condensation reaction 8. In order to better understand the function of each of the groups shown in FIG. 1, a set of postulated mechanisms 16, 18 and 20, for three different embodiments of condensation reaction 8, are shown in FIGS. 2, 3 and 4.

In FIGS. 2, 3 and 4, Nul is the protected phosphodiester 12 of condensation reaction 8, (Ion) is the same positive ion as in condensation reaction 8, Nu2 represents compound 14, and the final product shown is the protected phosphotriester 10.

In postulated mechanisms 16, 18 and 20, the dehydrating agent (Re) is mesitylene sulfonylchloride and CATSOL is N-methyl imidazole.

As seen in Part A of FIG. 2, the initial step of postulated mechanism 16 is the sulfonation of the tetrazole which occurs when the sulfur atom is nucleophilically attacked by the nitrogen of the tetrazole thereby causing the chloride group to leave and bond with the hydrogen which has left the tetrazole group. If CAT were a carboxylic acid, the nucleophilic attack would proceed by the sulfur atom being nucleophilically attacked by the oxygen anion of the carboxylic acid, thereby displacing a chloride anion. Thus, Part A of postulated mechanism 16 apparently will proceed if CAT is any nucleophile which can attack, for example, the sulfur atom of the dehydrating agent Re where Re is a sulfonylchloride or a similar sulfonyl derivative. Part A of postulated mechanisms 16, 18 and 20 will also function if Re is a carbodiimide although the nature of the reaction mechanism is as yet unclear.

It is thought that CATSOL which is N-methyl imidazole in each of postulated mechanisms 16, 18 and 20, functions to enhance the rate of the initial sulfonation of the tetrazole by first nucleophilically attacking the sulfur atom of the aryl sulfonylchloride as shown, and then by allowing the aryl sulfonyl group to transfer to the catalytic moiety (CAT). It is hypothesized that the initial sulfonation of CAT is the rate limiting step of the reaction. Thus, CATSOL of reaction 8 can theoretically be any group which enhances the initial sulfonation of CAT by bonding the aryl sulfonyl group, or any other dehydrating agent group, and thereafter allowing this group to transfer from CATSOL and bond to CAT.

In Part B of postulated mechanism 16, the O anion of the 3' phosphate of phosphodiester 12, attacks the sulfur atom of the sulfonyl group (which is now bonded to the tetrazole) causing the hydrogen of the HC to bond with the tetrazole and thereby form a chloride anion.

Thus, it can be seen that theoretically any of the dehydrating agent groups listed for FIG. 1, as well as other compounds which can function as electrophiles and accept nucleophilic attack from the O anion of the 3' phosphate of phosphodiester 12, can be used with this embodiment of condensation reaction 8.

Part C of postulated mechanism 16 shows the phosphorous atom of the 3' phosphate undergoing nucleophilic attack from the N 1 of the tetrazole, thereby causing the hydrogen of the tetrazole to leave and bond with the aryl sulfonyl leaving group, and also thereby forming the active cyclic intermediate seen in Part D.

In Part D of postulated mechanism 16, the active cyclic intermediate undergoes nucleophilic attack from the 5' hydroxyl group of compound 14 at the 3' phosphate phosphorous atom, thereby breaking the bond between the 1 N of the tetrazole and the phosphorous, and thereby forming an N-H bond with the hydrogen which has left the 5' hydroxyl of compound 14.

Thus, it is believed that any catalytic moiety/ catalytic alcohol, including but not limited to those listed for CAT and R-CAT in FIG. 1, which can form an active cyclic species which can in turn undergo nucleophilic attack from the 5' hydroxyl of compound 14, can function as the catalytic moiety/catalytic alcohol.

The embodiments of condensation reaction 8, shown in FIGS. 3 and 4 (postulated mechanisms 18 and 20), use 2-(2'-hydroxy phenyl) N-methyl imidazole and (2-hydroxy ethyl) N,N-dimethyl amine, respectively, as the diester bonded catalytic alcohols. As can be seen in Parts D of both postulated mechanisms 18 and 20, the active cyclic intermediates thought to be formed are similar to the active cyclic intermediate shown in postulated mechanism 16. All these active cyclic species are ring structures in which the 3' phosphorous, the ester oxygen, and portions of the catalytic alcohol form the cyclic ring. Other active cyclic intermediates would also have similar structures, for example, if the catalytic moiety were a properly positioned carboxylic acid, the cyclic species would be a mixed anhydride between the carboxylic acid and the phosphoric acid. The postulated mechanisms for all the various catalytic alcohols listed for FIG.1 are not shown as they are apparent in view of postulated mechanisms 16, 18 and 20.

Substituted or unsubstituted aromatic heterocycles and substituted or unsubstituted tertiary amines are both

nucleophiles and can therefore initially attack the sulfonyl electrophile as seen in Part A of postulated mechanisms 16, 18, and 20. Furthermore, these compounds have a structure such that if properly positioned they can form an active cyclic intermediate species similar to that shown in part D of postulated mechanisms 16, 18 and 20. These compounds are thus also suitable for use as the catalytic moiety.

Part E of postulated mechanism 16 shows the protected phosphotriester 10 which is formed by condensation reaction 8, shows the aryl sulfonylchloride group which results from reaction 8, and also shows the starting positive ion electrostatically bonded to the chloride anion.

It is postulated that in the three embodiments of condensation reaction 8, mechanistically shown in FIGS. 2, 3 and 4 (and other analagous embodiments of reaction 8 in which different catalytic moieties are used) the rate of formation of phosphotriester 10 is enhanced by the geometric positioning of the catalytic moiety relative to the 3' phosphate. This hypothesis leads to the conclusion that it is unimportant what the phosphate blocking group (R) is, providing it allows the catalytic moiety to position correctly relative to the 3' phosphate and providing it aids in preventing side reactions at the 3' phosphate. Accordingly, any such phosphate blocking group having the above-mentioned characteristics including, but not limited to, those described for FIG. 1 can theoretically be used. Phosphate blocking groups which can so position the

catalytic moieties include aryl, alkyl, cycloalkyl, and beta cyanoalkyl groups.

The 5' hydroxyl protecting group (Pr in condensation reaction 8) can be any group which can be bonded to the terminal 5' hydroxyl position of phosphodiester 12 and can prevent the terminal 5' hydroxyl from reacting with the 3' phosphate of the other phosphodiesters and other compounds in solution. Such 5' hydroxyl protecting groups include, for example, monomethoxytrityl and dimethoxytrityl.

Base, as shown in reaction 8 of FIG. 1, can be any nucleotide base. Thymine was the base used within the experiments described herein, because Thymine has no exocyclic nitrogens. Exocyclic nitrogens, present in other nucleotide bases, are nucleophilic and consequently participate in side reactions unless protected; typical protecting groups form amides with the base's exocyclic nitrogens.

Solvent, shown in reaction 8 of FIG. 1, can be any organic solvent or mixture of solvents capable of dissolving all of the reagents and compounds of reaction 8, and further capable of buffering the hydrochloric acid produced by reaction 8 -- HCl can be seen in Part B of postulated mechanism 16.

It can be seen that compound 14 can be any free or supported nucleoside, nucleotide, deoxynucleoside or deoxynucleotide, or oligonucleotide or deoxyoligonucleotide, or

diester or triester of any of these compounds, as long as the terminal 5' hydroxyl of any these compounds is deprotected.

It can also be seen from postulated mechanisms 16, 18 and 20 that although protected phosphotriester 10 is shown in FIG. 1 as a protected di-deoxynucleotide, compound 14 is shown as a supported deoxy-mononucleoside, and protected phosphodiester 12 is shown as a protected deoxy-mononucleotide diester, condensation reaction 8 can also form a di-ribonucleotide phosphotriester if compound 14 and protected phosphodiester 12 respectively represent a supported ribonucleoside and a protected ribonucleotide phosphodiester. Furthermore, condensation reaction 8 can form a protected oligonucleotide or deoxyoligonucleotide phosphotriester, if:

1) phosphodiester 12 is a di or oligo nucleotide phosphodiester or a di or oligo deoxynucleotide phosphodiester, wherein Pr represents a link to another nucleotide, deoxynucleotide, or chain of nucleotides or deoxynucleotides, or

2) compound 14 represents a chain of supported nucleotides or deoxynucleotides, or esters thereof, wherein the terminal 5' hydroxyl group of the chain is unprotected.

Rates of Condensation of Selected Catalytic Alcohols Compared with a Standard Para Chlorophenyl Blocking Group

In order to demonstrate that the positioning of the catalytic moieties resulted in rate enhancement, that CATSOL may aid in the initial sulfonation of CAT, and in order to show that the two preferred diester bonded catalytic alcohols provided significant rate enhancement, a series of experiments were performed as follows.

0.05 M 5'-Dimethoxytrityl-3'-(O-(5-tetrazoyl) phenyl phosphate) Thymidine (hereinafter the ortho tetrazole diester), 0.15 M mesitylene sulfonyl chloride, and 0.45 M N-methyl imidazole, were all dissolved in pyridine and the solution was mixed with silica gel bearing a 3' carboxyl linked Thymidine nucleoside. The silica gel was loaded to approximately twenty micromoles of Thymidine per gram of silica and a large excess of about 100 equivalents over the Thymidine bound to the silica gel, of the ortho tetrazole diester was used. The reaction was quenched at various times with a 9 to 1 v/v of acetonitrile/water and the dimethoxytrityl group was removed with 0.1 M P-TSA/$CH_3CN$. After extensive washings, the product was cleaved from the support with concentrated ammonium hydroxide, and the unreacted starting Thymidine was quantified by reverse phase high performance liquid chromatography. The results are summarized in line A of FIG. 5 wherein the percentage of the unreacted Thymidine is plotted against time to show the relative amount of rate enhancement.

The same experiment was performed with 2-(2'-hydroxyphenyl) N-methyl imidazole as the catalytic alcohol, (which yielded the ortho imidazole diester) and the results are summarized in line C of FIG. 5.

The experiment was then performed using the ortho tetrazole diester, but without adding any extra N-methyl imidazole to the solution. The results are summarized in line B of FIG. 5. The same experiment was then performed with a

para chlorophenyl blocking group bonded at the 3' phosphate, with the extra N-methyl imidazole in solution. The results are summarized in line D of FIG. 5. The experiment was then performed, using 5-(4'-hydroxyphenyl) tetrazole as the catalytic alcohol (which yields a para positioned tetrazole catalytic moiety following condensation with Thymidine) with extra N-methyl imidazole in solution. The results are summarized at line E of FIG. 5.

With respect to lines A and B, one can see the rate enhancement which results from adding extra N-methyl imidazole to the solution. This rate enhancement is consistent with the mechanism shown in inserts F of postulated mechanism 16.

Comparison of Line D (wherein the para chlorophenyl group was diester bonded to the 3' phosphate) with lines A, B or C (wherein the phosphodiesters were either the ortho tetrazole diester or the ortho imidazole diester) shows a much slower rate of reaction when using the para chlorophenyl group. This slower rate indicates that the para chlorophenyl group is likely not forming a single active cyclic intermediate species with the 3' phosphate phosphorous, as postulated. As will be discussed later, the para chlorophenyl group may be forming multiple acyclic species.

Line C of FIG. 5 shows that when using the ortho imidazole diester there is significant rate enhancement. However the rate is slightly slower than the rate observed

when using the ortho tetrazole diester. It is possible that tetrazole provides slightly greater rate enhancement than N-methyl imidazole because (as can be seen in Parts D of postulated mechanisms 16 and 18) the postulated cyclic intermediate species formed by the N-methyl imidazole catalytic moiety contains a positive charge on the N atom at which the methyl is attached, whereas tetrazole has a neutral cyclic intermediate with all uncharged nitrogens. Thus, it may be that when using N-methyl imidazole as the catalytic moiety there is a charge separation created in the postulated cyclic intermediate and, because charge separation is an endothermic process, creating the separation draws energy from the condensation reaction and results in a slightly slower condensation reaction than is observed when tetrazole is used as the catalytic moiety. However, despite a slightly slower rate when synthesizing dimers, N-methyl imidazole is the preferred catalytic moiety when synthesizing long chain nucleotides, as discussed below.

Line E shows that the rate of reaction when using a para positioned catalytic moiety is about the same as the reaction rate when using the para chlorophenyl diester. This experimentally demonstrates that it is the positioning of the catalytic moiety, and in the described embodiments of the invention wherein the catalytic moiety is linked to an aryl group, that it is the ortho positioning of the catalytic moiety which results in the rate enhancement. Moving the tetrazole from the ortho to the para position is, as is well-known, an electronically neutral move. The rate enhancement observed

with the 5-(2'-hydroxyphenyl) tetrazole catalytic alcohol is therefore most likely not due to any electronic changes in the blocking group or in the catalytic moiety; rather it is more likely due to the tetrazole being positioned relative to the 3' phosphate such that neighboring group participation can occur and the postulated active cyclic species can form.

In another set of experiments, using the foregoing experimental procedure, a determination was made of the reactivity of a nucleotide diester wherein 1-Methyl-2-(4'-hydroxyphenyl) imidazole was the diester bonded catalytic alcohol. This catalytic alcohol yields a para positioned N-methyl imidazole catalytic moiety upon condensation with a nucleotide.

It was observed the para positioned N-methyl imidazole diester yielded a condensation reaction rate comparable to the rate achieved with the standard para chlorophenyl diester. These results further indicate that in the embodiments in which the catalytic moiety is linked to a phenyl group, the ortho positioning of the catalytic moiety likely allows neighboring group participation which thereby enhances the rate of the nucleotide condensation reaction.

In this same set of experiments it was also shown that when using the ortho imidazole diester, deleting N-methyl imidazole from the reaction solution did not significantly decrease the rate of the condensation reaction.

Before the condensation reaction rates for the N-methyl imidazole and the tetrazole catalytic moieties were compared with the condensation reaction rate for the standard para chlorophenyl group, the inventors first made an initial determination (in order to save the time and trouble of running silica gel high performance liquid chromatography with all the previously listed catalytic alcohols) as to which catalytic alcohols showed significant rate enhancement.

Initial Determination of Reactivity

Dimethoxytrityl protected Thymidine monomer, with various catalytic moieties diester bonded thereto, was reacted with the hydroxyl group of an alcohol flourescently labeled with a danzyl fluorophore in order to triester link the danzyl alcohol group to the protected phosphodiester. This triester link can be seen in FIG. 6. Danzyl alcohol flouresces upon irradiation with ultraviolet light and a qualitative assessment of the amount of product triester was made by visual observation of the flourescence of the triester upon irradiation with a long wave ultraviolet light. The hydroxyl group of danzyl alcohol reacts with the diester analogously to the reaction of a terminal 5' hydroxyl group of a nucleotide or a nucleoside, and therefore, based on the amount of flourescence observed, estimates of the relative reactivities of the various catalytic alcohols were made by the observer. The full experimental follows below.

1. Synthesis of Danzyl Alcohol

270 mg (1 millimole) of danzyl chloride and 61 mg (1 millimole) of ethanol amine were dissolved in 5 ml of

pyridine. After one hour, the reaction was quenched with 50 (1 millimole) of ethanol amine were dissolved in 5 ml of pyridine. After one hour, the reaction was quenched with 50 microliters of water, and the resulting mixture was evaporated to a gum which was dissolved in methylene chloride and then extracted against a saturated sodium chloride solution. The organic layer was then evaporated and purified on preparative thin layer chromatography plates, using diethylether as the eluant.

2. Preparation of Danzyl Alcohol Triesters and Reactivity Assessments

Following preparation of the danzyl alcohol it was reacted with the para chlorophenyl, the 8-hydroxy quinoline, the (2-hydroxy ethyl) N,N,dimethyl amine, the (3-hydroxy propyl) N,N-dimethyl amine, the 2-(hydroxy methyl) pyridine, the 2-(2'-hydroxy ethyl) pyridine, the 2-(hydroxy methyl) N-methyl imidazole, the 2-(2'hydroxy ethyl) N-methyl imidazole, the 5-(2'-hydroxy ethyl) tetrazole, the 2-(2'-hydroxyphenyl) N-methyl imidazole and the 5-(2'-hydroxyphenyl) tetrazole diesters of 5' hydroxy protected Thymidine. The reactivity assessment for all the diesters and for the preparation of all the danzyl alcohol triesters are identical. Therefore, only the full experimental is given for reactivity comparison of the 5-(2'-hydroxyphenyl) tetrazole diester of dimethoxytrityl protected Thymidine compared with the para chlorophenyl diester. Similarly, only the full experimental is given for the preparation of the 5-(2'-hydroxyphenyl) tetrazole danzyl alcohol triester of Thymidine, and for the preparation of the para chlorophenyl danzyl alcohol triester of Thymidine.

3. Experimental for the Reactivity Comparison of 5-(2'-hydroxyphenyl) Tetrazole Diester with a para Chlorophenyl Diester, and for the Danzyl Alcohol Triester Preparation of the Same

10 mg of the ortho tetrazole diester and 10 mg of the para chlorophenyl protected diester were each combined with 6 microliters of N-methyl imidazole, 0.1 mg of danzyl alcohol, and 0.5 ml of pyridine. The solution was evaporated to a gum and redissolved in 400 microliters of dry pyridine. To this solution was added 32 mg of mesitylene sulfonylchloride in 400 microliters of dry pyridine. After 30 minutes the reaction was quenched with 10 microliters of water and 3 microliters of the reaction mixture were analyzed by thin layer chromatography, first by eluting with ether and then with 10% MeOH in chloroform. The amount of the product triester was measured by estimating the amount of flourescence, as previously described.

4. Reactivity Comparison of Various Catalytic Alcohol Diesters with a Para-Chlorophenyl Group Diester

The relative rate of reactivity for all of the various phosphotriester formation catalyst/blocking groups listed for R-CAT in FIG. 1 were thereafter determined by the above-described method; these relative rates are listed below.

Table 1

Relative Reactivity Rates of the Coupling of Various Phosphodiesters With Danzyl Alcohol

| Catalytic Alcohol | Approximate reactivity rate compared with para chlorophenyl |
|---|---|
| a) 8-hydroxy quinoline | Less than 5 |
| b) (2-hydroxy ethyl)N,N,-dimethyl amine | Less than 5 |
| c) (3-hydroxy propyl)N,N-dimethyl amine | Less than 5 |
| d) 2-(hydroxy methyl)pyridine | Less than 5 |
| e) 2-(2'-hydroxy ethyl)pyridine | Less than 5 |
| f) 2-(2'-hydroxy ethyl)N-methyl imidazole | About 10 |
| g) 2-(hydroxy methyl)N-methyl imidazole | Unstable diester |
| h) 5-(2'-hydroxy ethyl)tetrazole | About 10 |
| i) 5-(2'-hydroxyphenyl)tetrazole | About 100 |
| j) 2-(2'-hydroxyphenyl)N-methyl imidazole | About 20 |
| k) para chlorophenyl | 1.0 |

After it was observed that 5-(2'hydroxyphenyl) tetrazole and 2-(2'-hydroxyphenyl) N-methyl imidazole catalytic alcohols gave the greatest rate enhancement, the full silica gel column high performance liquid chromatography experimental (as described previously) was done for these two ortho diesters.

5. Extrapolations from these Experiments

As mentioned previously, Thymidine was the nucleotide used in these experiments because Thymidine has no exocyclic nitrogen which require protection as amides. As will be apparent to those skilled in the art, the same experimentals for reactivity assessment, and the same reactions shown in FIGS. 1 and 6, could be performed with phosphodiesters of other deoxynucleotides such as Adenine, Guanidine and Cytidine or other ribonucleotides, such as Uridine (with the 2' hydroxyl group blocked), other chains of ribonucleotides (with blocked 2' hydroxyl groups), or other chains of deoxynucleotides. Using different nucleotides, or different ribonucleotides, would not alter the reaction mechanisms (which are thought to be either the same or analagous to postulated mechanisms 16, 18 and 20), and the same relative reactivity rates with the various diester bonded catalytic alcohols listed previously would theoretically result.

Experimental NMR Data Supporting the Postulated Structure of the Active Cyclic Intermediates

EXPERIMENTAL A

The ortho tetrazole diester of dimethoxytrityl protected Thymidine (0.2 M), and N-methyl imidazole (0.8M) were dissolved in excess pyridine and the reaction was allowed to proceed. Thereafter the P31 NMR spectra was taken and the results are seen in Line A of FIG. 7. The external standard peak shown in FIG. 7 is phosphoric acid. No aryl sulfonylchloride was in the solution, nor was any other dehydrating agent present, and the postulated active cyclic intermediate shown in FIG. 2 apparently did not form.

EXPERIMENTAL B

Thereafter, using the same proportions of the same ingredients above, excess mesitylene sulfonylchloride (0.4 M) was added to the product reaction mixture of Experimental A and the P31 NMR results are shown in Line B of FIG. 7. It is believed that the mesitylene sulfonylchloride acts as a dehydrating agent and allows the postulated reaction mechanism 16 to proceed and to form the active cyclic species of Part D.

The new NMR peak, which is the singlet seen in Line B of FIG. 7, suggests that a new compound has indeed been formed. Although not shown in FIG. 7, the new peak in Line B is located -20.4209 parts per million frequency difference from the phosphoric acid standard.

EXPERIMENTAL C

Thereafter, using the same proportions of the same ingredients as in Experimental B above, excess water was added to the reaction mixture of Experimental B and the P31 NMR results are seen in Line C of FIG. 7. The peak seen in Line C of FIG. 7 is the same peak as the peak seen in Line A of FIG. 7, indicating that the electrons on the hydroxyl group of water have likely nucleophilically attacked the phosphorus nucleus

of the postulated active cyclic intermediate (in a similar fashion to the attack seen in Part D of FIG. 2) thus causing reversion to the starting dimethoxytrityl protected ortho tetrazole diester. The existence of the postulated active cyclic intermediate and steps D and E of FIG. 2 are, therefore, consistent with these experiments.

## EXPERIMENTAL D

In a set of experiments similar to those outlined in Experimentals A, B and C above, the para chlorophenyl diester of dimethoxytrityl protected Thymidine (0.2M) was reacted with N-methyl imidazole (0.8M) and all were dissolved in pyridine. The P31 NMR spectral peak is seen in line A of FIG. 8. This peak reflects the reaction product without the addition of arylsulfonyl chloride or any other dehydrating agent. The hypothesis is that there is no initial nucleophilic attack of the sulfur atom as seen in insert F of FIGS. 2, 3 and 4, and thus, that no intermediate species formed.

Thereafter excess, 0.4 M, mesitylene sulfonyl chloride was added to the reaction mixture and the resulting reaction formed the P31 NMR peaks shown in line B of FIG. 8. Line B shows that a complex, multi-peak product is formed by the product of para chlorophenyl phosphodiester activated with mesitylene sulfonylchloride. This peak indicates the nonexistence of a single, active, cyclic intermediate species, as is indicated by the single peak in Line B of FIG. 7, and rather indicates that the active species in the solution is

many different intermediates. The nonexistence of the postulated single active cyclic intermediate species when using the para chlorophenyl as the catalytic moiety/blocking group, is thought to be the reason for the relatively low rate of the nucleotide condensation reaction.

Again, postulated mechanisms 16, 18 and 20 are experimentally suggested, as is the existence of the single active intermediate species shown in Part D of FIGS. 2, 3 and 4.

Line C of FIG. 8 shows the P31 NMR peak of the product of Experimental D, after the addition of mesitylene sulfonylchloride, and further after the addition of water which quenches the reaction. The water is believed to cause reversion to the starting material through nucleophilic attack by the hydroxyl group on all of the intermediates formed. The P31 NMR peak shown, which is the same peak seen in line A, indicates that all the intermediates formed by the addition of mesitylene sulfonyl chloride have reverted to the starting para chlorophenyl diester.

Experimental for Synthesis of Deoxyoligothymidine

The synthesis of an oligonucleotide containing eight Thymidines was undertaken to show the use of the preferred catalytic alcohols in a sequential oligonucleotide synthesis.

To perform the synthesis, 50 mg of silica was first loaded with one micromole of Thymidine in order to form an

insoluble support for the sequential elongation of the chain. Two separate experiments were performed, using first one, and then the other, of both the ortho imidazole diester and the ortho tetrazole diester of thymidine. The thymidine diesters were added one at a time, with each cycle consisting of:

(a) Deblocking the dimethoxytrityl group using 5 ml of 2.5% dichloroacetic acid in dichloromethane and reacting for two minutes;

(b) Washing the silica with 10% pyridine/90% acetonitrite;

(c) Coupling the Thymidine monomer diester to the support using 600 microliters of a pyridine solution which was, 0.15 M in mesitylene chloride, 0.45 M in N-methyl imidazole, and 0.05 M in the dimethoxytrityl protected diester (this reaction was performed for 5 minutes);

(d) Washing the silica with 10% n-butanol in acetonitrite; and

(e) Washing the silica with 10% pyridine/90% acetonitrite.

This cycle was repeated seven times to yield a protected deoxy-Thymidine 8 derivative, covalently linked to the silica support.

The silica gel column was again treated with dichloroacetic acid to remove the dimethoxytrityl group, washed with methanol, and then treated with concentrated ammonium hydroxide for two hours to cleave the deoxy-Thymidine octamer from the silica gel.

The resulting mixture was centrifuged and the supernatant was evaporated. This residue was dissolved in a one

to one (v/v) dioxane-water solution and the resulting solution was then made 0.3 M in tetramethyl guanidine and ortho nitrophenyl aldoxime; thereafter the resulting solution was heated to 60°C for 12 hours, thus cleaving the catalytic alcohol groups from the deoxy-Thymidine octamer and resulting in only nucleotide-nucleotide diester linkages. This solution was then neutralized with acetic acid and extracted three times with ether.

A small portion (about 1%) of the aqueous phase was analyzed by kination and gel electrophoresis. The kination was performed using T4 kinase and gamma labelled ATP under standard conditions. The kinased reaction was electrophoresed on a 7 M urea 20% acrylamide gel. X-ray film was placed on the gel and the autoradiogram seen in FIG. 9 was obtained.

Referring to FIG. 9, lane A is a deoxy-Thymidine 20 standard, which was synthesized by standard phosphite chemistry, and contains traces of all nucleotide oligomers from T2 to T20. Lane B is the product of the T8 synthesis (described in the preceding experimental) using the ortho imidazole diester. Lane C is the product of the T8 synthesis described in the preceding experimental, using the ortho tetrazole diester. As clearly shown, T8 was the dominant product of both test syntheses using either diester bonded catalytic alcohol.

In a set of further experiments, the ortho imidazole diester was used for the synthesizing a pentadecathymidylic acid (a chain of 15 thymidines). The ortho imidazole diester

was used with these experiments because it is somewhat easier to make this diester than to make the ortho tetrazole diester, and it also provided consistent results when forming longer chain nucleotides. These experiments were performed analogously to those described for Thymidine 8 synthesis. The condensation reaction was carried out for 5 minutes and the forming nucleotide chain was sequentially deprotected with 2.5 percent DCA/$CH_2Cl_2$. Aldoxime treatment and high performance liquid chromatography analysis demonstrated that the ortho imidazole diester gave superior results when preparing a long chain nucleotide as compared with a standard para-chlorophenyl diester.

Although not seen in FIG. 9, the use of the ortho tetrazole diester and the ortho imidazole diester results in a greater purity of oligonucleotide yield (especially if nucleotides other than Thymidine are used) than would be obtained if standard para chlorophenyl blocking groups were used. This greater purity results because the condensation of each monomer proceeds faster, due to the presence of the catalytic moieties, and therefore the rate of the side reactions is not competitive with the condensation reaction.

Experimental for the Preparation of Phosphodiesters With Various Catalytic Alcohols Diester Bonded Thereto

1. Preparation of 5'-Dimethoxytrityl-3'-(o-(5-tetrazoyl) phenyl phosphate) Thymidine:

To a solution of 1.2 ml (7.5 mmole) 2,4-dichlorophenyl phosphodichlorodate and 3.0 ml (25.0 mmole) 2,6-lutidine,

both in 50 ml anhydrous tetrahydrofuran, subsequently cooled to 0°C, was added a solution of 2.9 g (5.0 mmole) 5'-Dimethoxytrityl Thymidine (dried by co-evaporation from pyridine) in 25 ml pyridine, with stirring. After 30 minutes the resulting solution was brought to room temperature and added to 1.6 g (10.0 mmole) 5-(2'-hydroxyphenyl) tetrazole (dried by co-evaporation from acetonitrile/pyridine). After stirring at room temperature for two hours the product solution was evaporated to an oil, taken up in 50 ml methylene chloride, washed with .1 M triethylammonium bicarbonate (pH = 8, 2x50 ml), dried over anhydrous sodium sulfate and evaporated to a foam. A solution of this foam in 50 ml concentrated ammonium hydroxide/dioxane (1/1) was heated at 60°C for 12 hours. Following evaporation, the product diester was purified and isolated by silica gel column chromatography using 9 to 1 v/v acetonitrile/water. The product fractions were pooled, concentrated, and evaporated from 5 percent triethylamine/acetonitrile (2x50 ml) to yield the anhydrous triethylammonium salt of the diester.

2. Preparation of 5'-Dimethoxytrityl-3'-(o-(2-N-methyl imidazole) phenyl phosphate)Thymidine

To a solution of 1.2 ml (7.5 mmole) 2,4-dichlorophenyl phosphodichlorodate and 3.0 ml (25.0 mmole) 2,6-lutidine, both in 50 ml anhydrous tetrahydrofuran, subsequently cooled to 0°C, was added a solution of 2.9 g (5.0 mmole) 5'-Dimethoxytrityl Thymidine (dried by co-evaporation from pyridine) in 25 ml pyridine, with stirring. After 30 minutes the solution was brought to room temperature and added to 1.79 g (10.0 mmole)

2-(2'-hydroxyphenyl)N-methyl Imidazole (dried by co-evaporation from acetonitrile/pyridine). After stirring at room temperature for two hours the product solution was evaporated to an oil, taken up in 50 ml methylene chloride, washed with .1 M triethylammonium bicarbonate (pH = 8, 2x50 ml), dried over anhydrous sodium sulfate and evaporated to a foam. A solution of this foam in 50 ml concentrated ammonium hydroxide/dioxane (1/1) was heated at 60°C for 12 hours. Following evaporation, the product diester was purified and isolated by silica gel column chromatography using 9 to 1 v/v acetonitrile/water. The product fractions were pooled, concentrated and evaporated from 5 percent triethylamine/acetonitrile (2x50 ml) to yield the anhydrous triethylammonium salt of the diester.

3. Preparation of 5'-Dimethoxytrityl-3'-(3-N,N,dimethyl amino propyl phosphate)Thymidine

To a solution of 1.2 ml (7.5 mmole) 2,4-dichlorophenyl phosphodichlorodate and 3.0 ml (25.0 mmole) 2,6-lutidine, both in 50 ml anhydrous tetrahydrofuran, subsequently cooled to 0°C, was added a solution of 2.9 g (5.0 mmole) 5'-Dimethoxytrityl Thymidine (dried by co-evaporation from pyridine) in 25 ml pyridine, with stirring. After 30 minutes the solution was brought to room temperature and added to 1.0 g (10.0 mmole) 3-Hydroxypropyl-N,N-dimethylamine (dried by co-evaporation from acetonitrile/pyridine). After stirring at room temperature for two hours the product solution was evaporated to an oil, taken up in 50 ml methylene chloride, washed with .1 M triethylammonium bicarbonate (pH = 8, 2x50 ml), dried over anhydrous sodium sulfate and evaporated to a foam. A solution of this foam in 50

ml concentrated ammonium hydroxide/dioxane (1/1) was heated at 60°C for 12 hours. Following evaporation, the product diester was purified and isolated by silica gel column chromatography using 9 to 1, v/v, acetonitrile/water. The product fractions were pooled, concentrated and evaporated from 5 percent triethylamine/acetonitrile (2x50 ml) to yield the anhydrous triethylammonium salt of the diester.

As can be seen by these three experimentals, the preparations of all these phosphodiesters is done by the same process. The phosphodiesters of all the catalytic alcohols listed in Table 1 were also prepared by this process.

The following catalytic alcohol/blocking groups, used to prepare the corresponding phosphodiesters, were purchased commercially:

a) 8-hydroxy quinoline,

b) (2-hydroxy ethyl)N,N,dimethyl amine,

c) (3-hydroxy propyl)N,N-dimethyl amine,

d) 2-(hydroxy methyl)pyridine, and

e) 2-(2'-hydroxy ethyl)pyridine

The remaining starting catalytic alcohols listed in Table 1, which are:

f) 2-(2'-hydroxy ethyl)N-methyl imidazole,

g) 2-(hydroxy methyl)N-methyl imidazole,

h) 5-(2'-hydroxy ethyl)tetrazole,

i) 5-(2'-hydroxyphenyl)tetrazole, and

j) 2-(2'-hydroxyphenyl)N-methyl imidazole,

were made from a standard literature preparation, well known to those skilled in the art, references for which appear below. Roe, J. Chem. Soc. (1963) 2195; Finnegan, W. G., Henry, R. A., Lofquist, R., J. Am. Chem. Soc. (1958) 80, 3908; G. A. Rogers, T. C. Bruiie, J. Am. Chem. Soc. (1974) 96, 2463.

Methods of preparing other phosphotriesters with other catalytic alcohols diester bonded thereto will be apparent to those skilled in the art in view of the phosphodiesters and the preparation methods disclosed above.

It should be understood, that in addition to the variations of the invention described in the specifications, other modifications and variations of the invention will be obvious to those skilled in the art. The terms and expressions as used herein are terms of description only and not of limitation, the invention being limited only by the scope of the claims which follow.

CLAIMS

1. A catalytic alcohol diester bonded to the 3' phosphate of a nucleotide.

2. An internucleotide condensation catalytic moiety bonded to a blocking group, said blocking group being bonded to the 3' phosphate of a nucleotide.

3. A compound comprising a blocking group diester bonded to the 3' phosphate of a nucleotide wherein said blocking group has a catalytic moiety bonded thereto such that neighboring group participation can occur during formation of a phosphotriester.

4. A compound comprising a blocking group diester bonded to the 3' phosphate of a nucleotide wherein said blocking group has a catalytic moiety bonded thereto such that an active cyclic species involving said catalytic moiety and the 3' phosphorous of said nucleotide can form, said active cyclic species capable of undergoing nucleophilic attack from the terminal 5' hydroxyl group of a nucleotide or nucleoside, said nucleophilic attack resulting in the formation of a phosphotriester.

5. The compound of any of Claims 2, 3, or 4 wherein said blocking group is a substituted or unsubstituted, aryl, alkyl, cycloalkyl, or beta cyanoalkyl group.

6. The compound of Claim 5, wherein the catalytic moiety is a substituted or unsubstituted, aromatic nitrogen heterocycle, tertiary amine, or carboxylic acid.

7. The compound of any of Claims 2, 3, 4, 5 or 6 wherein the catalytic moiety is substituted or unsubstituted, quinoline, pyridine, imidazole, or tetrazole.

8. The compound of Claim 1 wherein said catalytic alcohol is 8-hydroxy quinoline, (2-hydroxy ethyl) N,N,dimethyl amine, (3-hydroxy propyl) N,N-dimethyl amine, 2-(hydroxy methyl) pyridine, 2-(2'-hydroxy ethyl) pyridine, 2-(hydroxy methyl) N-methyl imidazole, 2-(2'-hydroxy ethyl) N-methyl imidazole, 5-(2'-hydroxy ethyl) tetrazole, 5-(2'-hydroxyphenyl) tetrazole, or 2-(2'-hydroxyphenyl) N-methyl imidazole.

9. An intermediate compound formed when using the compound of Claim 1 in an internucleotide condensation reaction, said intermediate compound being capable of undergoing nucleophilic attack from the 5' hydroxyl of a nucleotide or nucleoside.

10. The compound of Claim 9 having the formula,

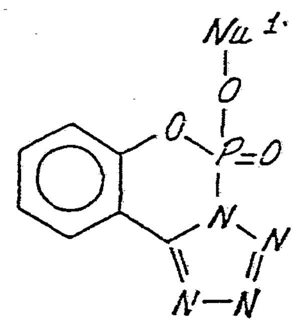

wherein Nul represents a nucleotide, the compound being formed during a condensation reaction between a nucleoside or a second nucleotide and said nucleotide having 5-(2'hydroxylphenyl) tetrazole diester bonded to its 3' phosphate position.

11. A method of oligonucleotide synthesis comprising the use of the compound of any of Claims 1, 2, 3, 4, 7 or 8.

. The method of Claim 11 wherein the internucleotide condensation rate is further enhanced by the addition of CATSOL.

. A method of enhancing the rate of an internucleotide condensation reaction comprising:

a) protecting the 5' hydroxyl group of the compound of any of Claims 2, 3, 4, 7 or 8 thereby forming a protected phosphodiester;
b) ionizing at least one of the hydroxyl groups linked to the 3' phosphate of said phosphodiester thereby forming an oxygen anion;
c) electrostatically bonding a positive ion to said oxygen anino thereby forming a salt;
d) dissolving a dehydrating agent, said salt, and CATSOL in an organic solvent thereby forming an organic solution; and
e) contacting said organic solution with a nucleoside or a nucleotide thereby forming a phosphotriester.

4. The method of Claim 13 further comprising:

a) cleaving said phosphotriester from said support;
b) deprotecting said 5' hydroxyl group; and
c) removing the blocking group and the catalytic moiety.

CLAIMS

1. The use of a catalytic alcohol diester bonded to the 3' phosphate of a nucleotide in internucleotide condensation.

2. The use in internucleotide condensation of an internucleotide condensation catalytic moiety bonded to a blocking group, said blocking group being bonded to the 3' phosphate of a nucleotide.

3. The use in internucleotide condensation of a compound comprising a blocking group diester bonded to the 3' phosphate of a nucleotide wherein said blocking group has a catalytic moiety bonded thereto such that neighboring group participation can occur during formation of a phosphotriester.

4. The use in internucleotide condensation of a compound comprising a blocking group diester bonded to the 3' phosphate of a nucleotide wherein said blocking group has a catalytic moiety bonded thereto such that an active cyclic species involving said catalytic moiety and the 3' phosphorous of said nucleotide can form, said active cyclic species capable of undergoing nucleophilic attack from the terminal 5' hydroxyl group of a nucleotide or nucleoside, said nucleophilic attack resulting in the formation of a phosphotriester.

5. The method of any of Claims 2, 3 and 4 wherein said blocking group is a substituted or unsubstituted, aryl, alkyl, cycloalkyl, or beta cyanoalkyl group.

6. The method of Claim 5, wherein the catalytic moiety is a substituted or unsubstituted, aromatic nitrogen heterocycle, tertiary amine, or carboxylic acid.

7. The method of any of Claims 2, 3, 4, 5 and 6 wherein the catalytic moiety is substituted or unsubstituted, quinoline, pyridine, imidazole, or tetrazole.

8. The method of Claim 1 wherein said catalytic alcohol is 8-hydroxy quinoline, (2-hydroxy ethyl) N,N,dimethyl amine, (3-hydroxy propyl) N,N-dimethyl amine, 2-(hydroxy methyl) pyridine, 2-(2'-hydroxy ethyl) pyridine, 2-(hydroxy methyl) N-methyl imidazole, 2-(2'-hydroxy ethyl) N-methyl imidazole, 5-(2'-hydroxy ethyl) tetrazole, 5-(2'-hydroxyphenyl) tetrazole, or 2-(2'-hydroxyphenyl) N-methyl imidazole.

9. A method of claim 1 wherein an intermediate compound is formed when using the compound of Claim 1 in an internucleotide condensation reaction, said intermediate compound being capable of undergoing nucleophilic attack from the 5' hydroxyl of a nucleotide or nucleoside.

10. A method of claim 9 wherein the intermediate compound has the formula:

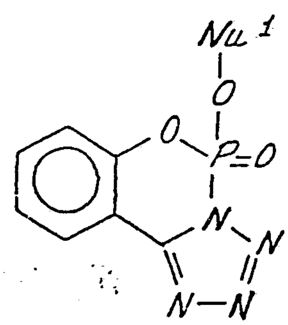

wherein Nu1 represents a nucleotide, the compound being formed during a condensation reaction between a nucleoside or a second nucleotide and said nucleotide having 5-(2'hydroxylphenyl) tetrazole diester bonded to its 3' phosphate position.

11. A method of any one of the preceding claims wherein the internucleotide condensation rate is further enhanced by the addition of CATSOL.

12. A method of enhancing the rate of an internucleotide condensation reaction comprising:

a) protecting the 5' hydroxyl group of the compound of any of Claims 1 to 10 thereby forming a protected phosphodiester;
b) ionizing at least one of the hydroxyl groups linked to the 3' phosphate of said phosphodiester thereby forming an oxygen anion;
c) electrostatically bonding a positive ion to said oxygen anino thereby forming a salt;
d) dissolving a dehydrating agent, said salt, and CATSOL in an organic solvent thereby forming an organic solution; and
e) contacting said organic solution with a nucleoside or a nucleotide thereby forming a phosphotriester.

13. The method of Claim 12 further comprising:

a) cleaving said phosphotriester from said support;
b) deprotecting said 5' hydroxyl group; and
c) removing the blocking group and the catalytic moiety.

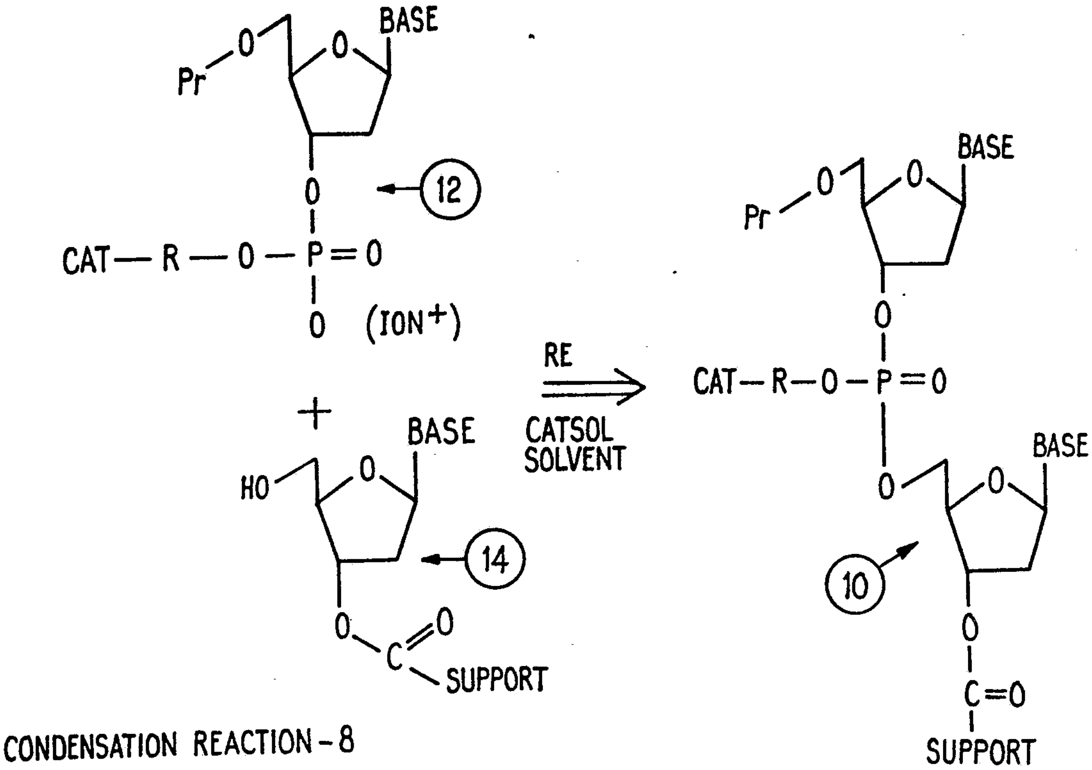


CONDENSATION REACTION - 8

FIG.1

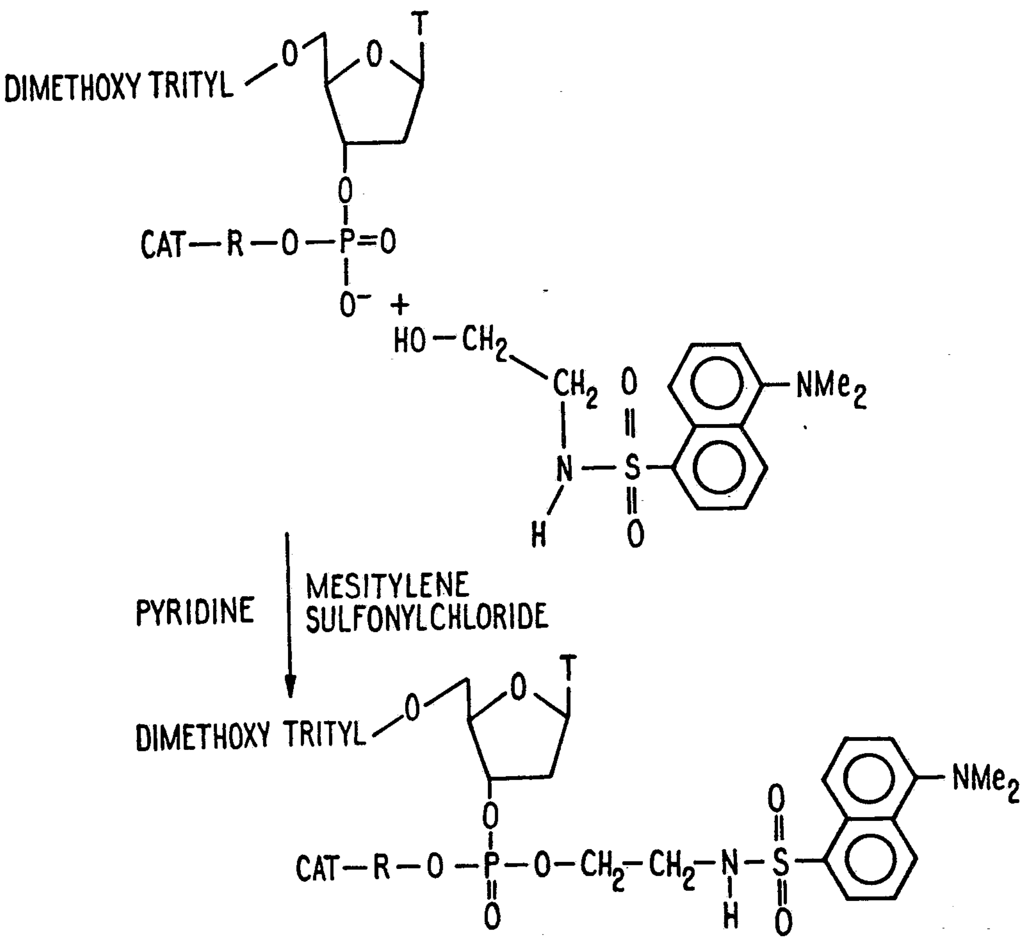


FIG.6

# FIG.2

2 HYDROXYPHENYL ( 5 TETRAZOLE ) CATALYST
MECHANISM 16

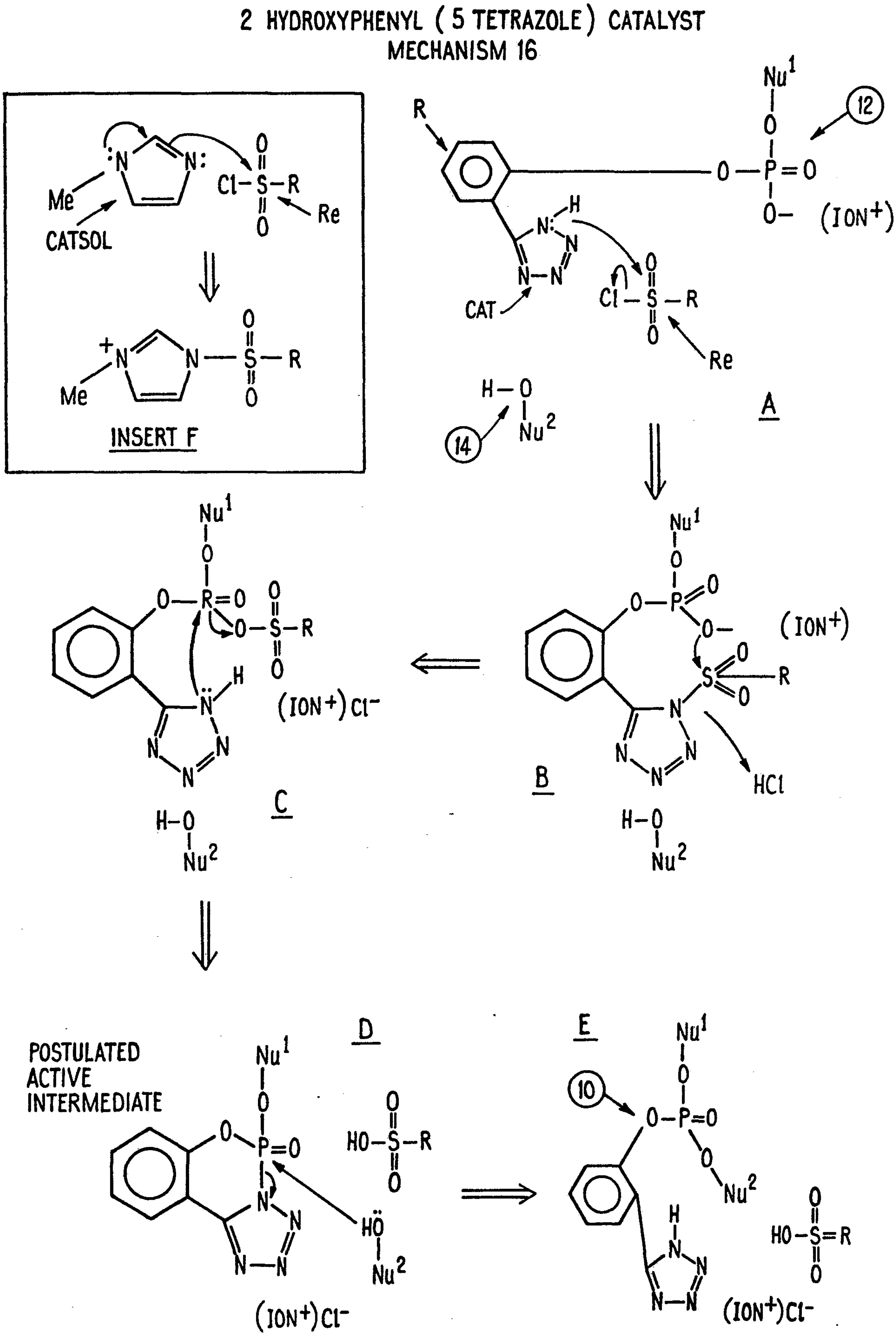

# FIG.3

2 HYDROXYPHENYL (2 N-METHYL IMIDAZOLE) CATALYST
MECHANISM 18

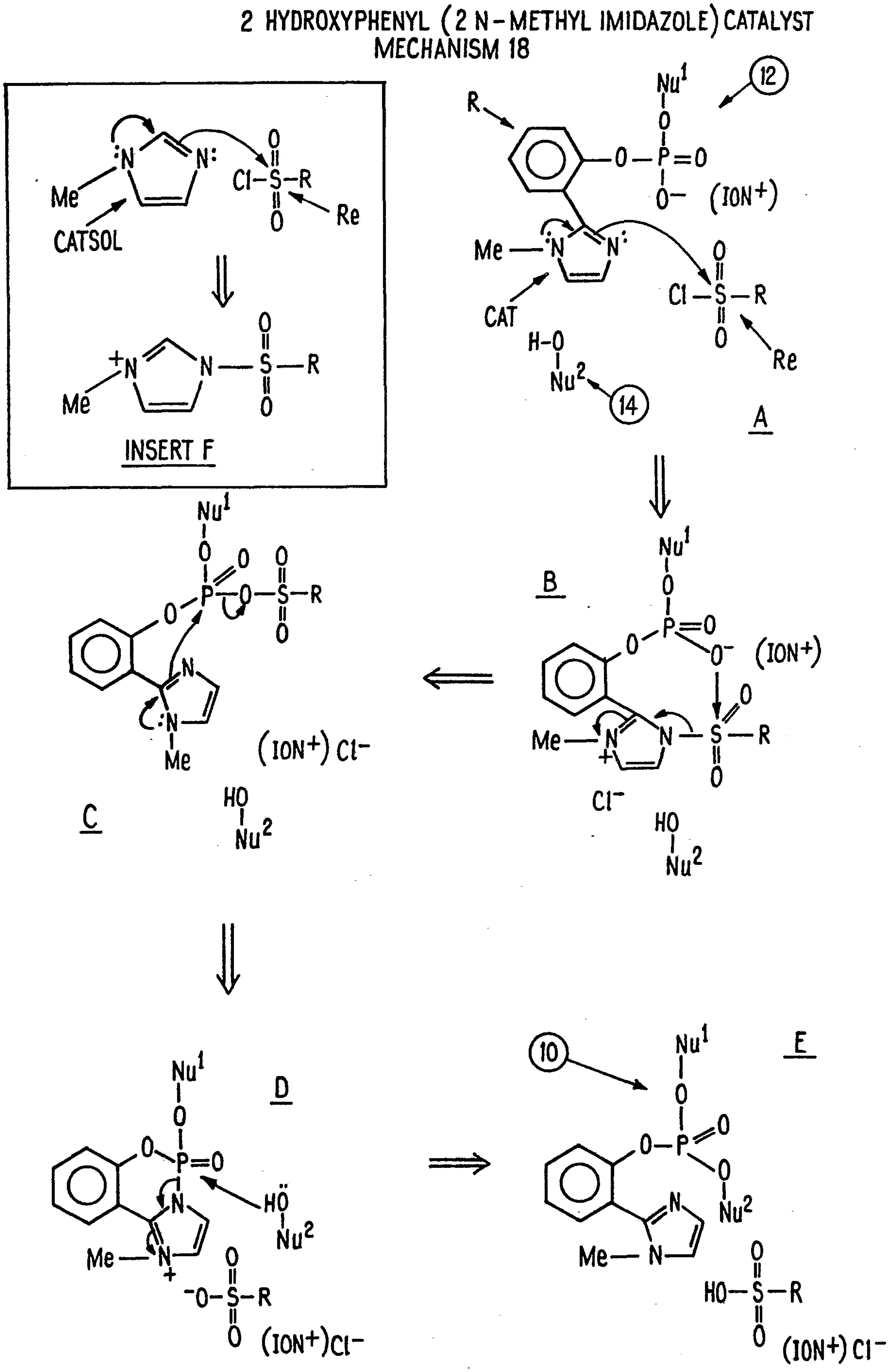

INSERT F

A

B

C

D

E

# FIG.4

(2 HYDROXYPHENYL) N N DIMETHYL AMINE CATALYST
MECHANISM 20

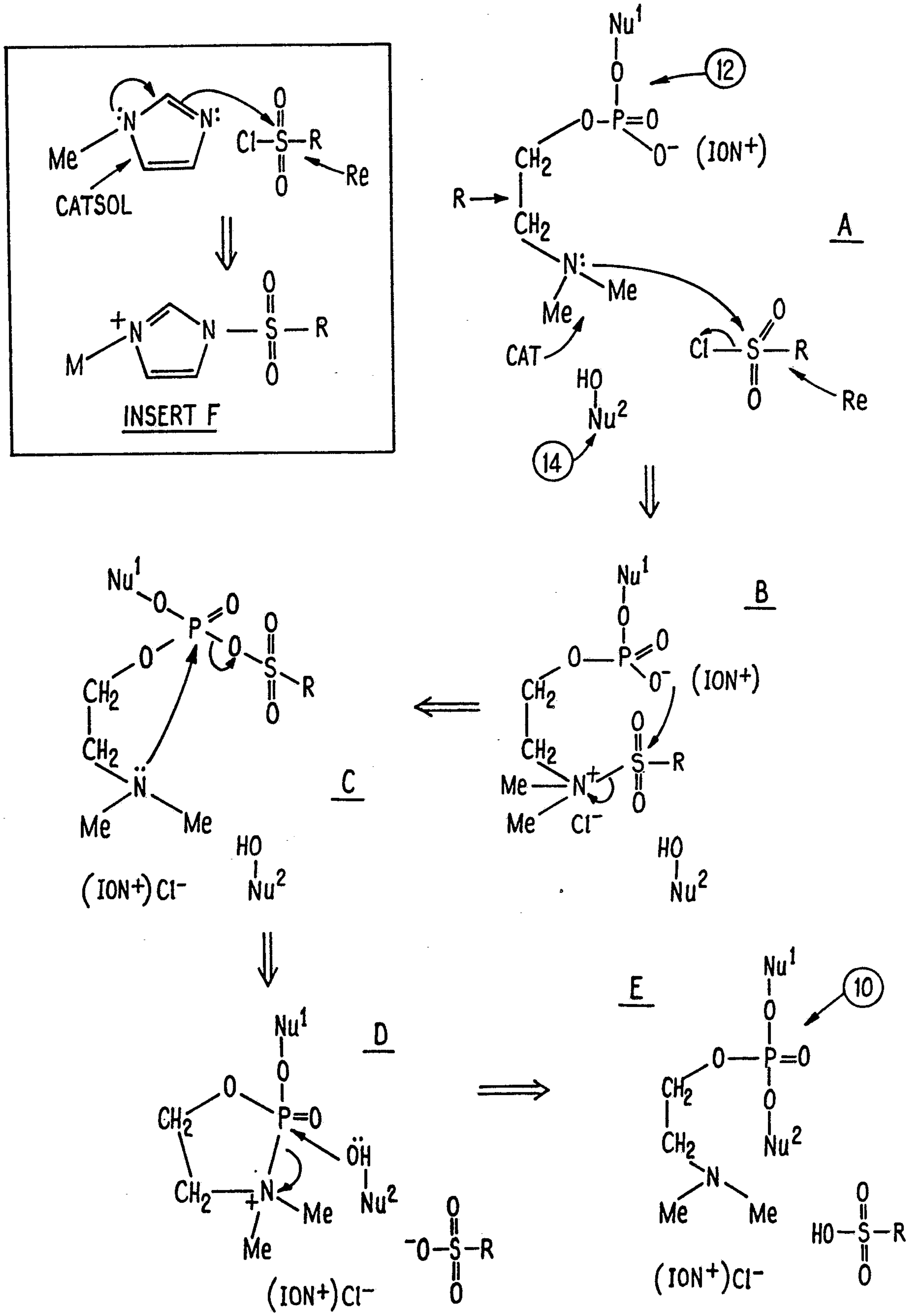

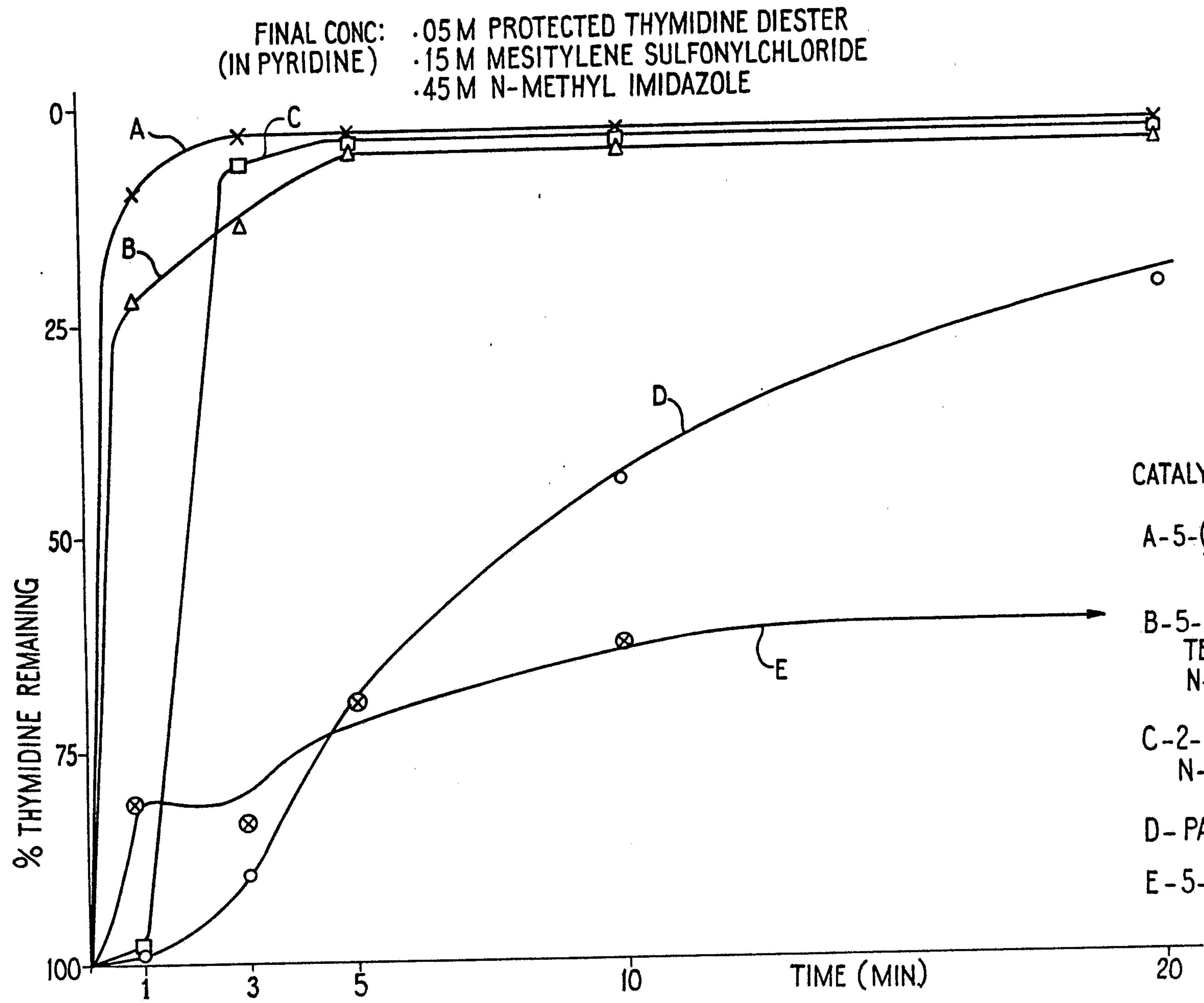


FIG.5

CATALYTIC ALCOHOLS (R-CAT)

A-5-(2'-HYDROXY PHENYL) TETRAZOLE

B-5-(2'-HYDROXY PHENYL) TETRAZOLE W/O EXTRA N-METHYL IMIDAZOLE

C-2-(2'-HYDROXY PHENYL) N-METHYL IMIDAZOLE

D- PARA CHLOROPHENYL

E-5-(4' HYDROXY PHENYL) TETRAZOLE

# FIG.7

*

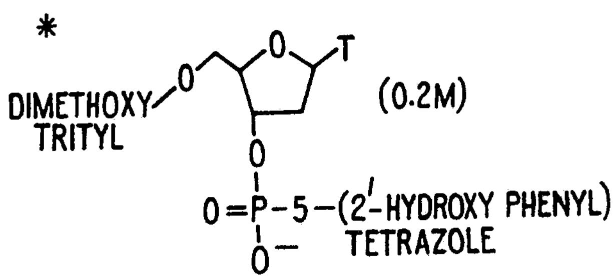

(0.2M)

+

N-METHYL IMIDAZOLE (0.8M)

PYRIDINE

TO LINE (A)

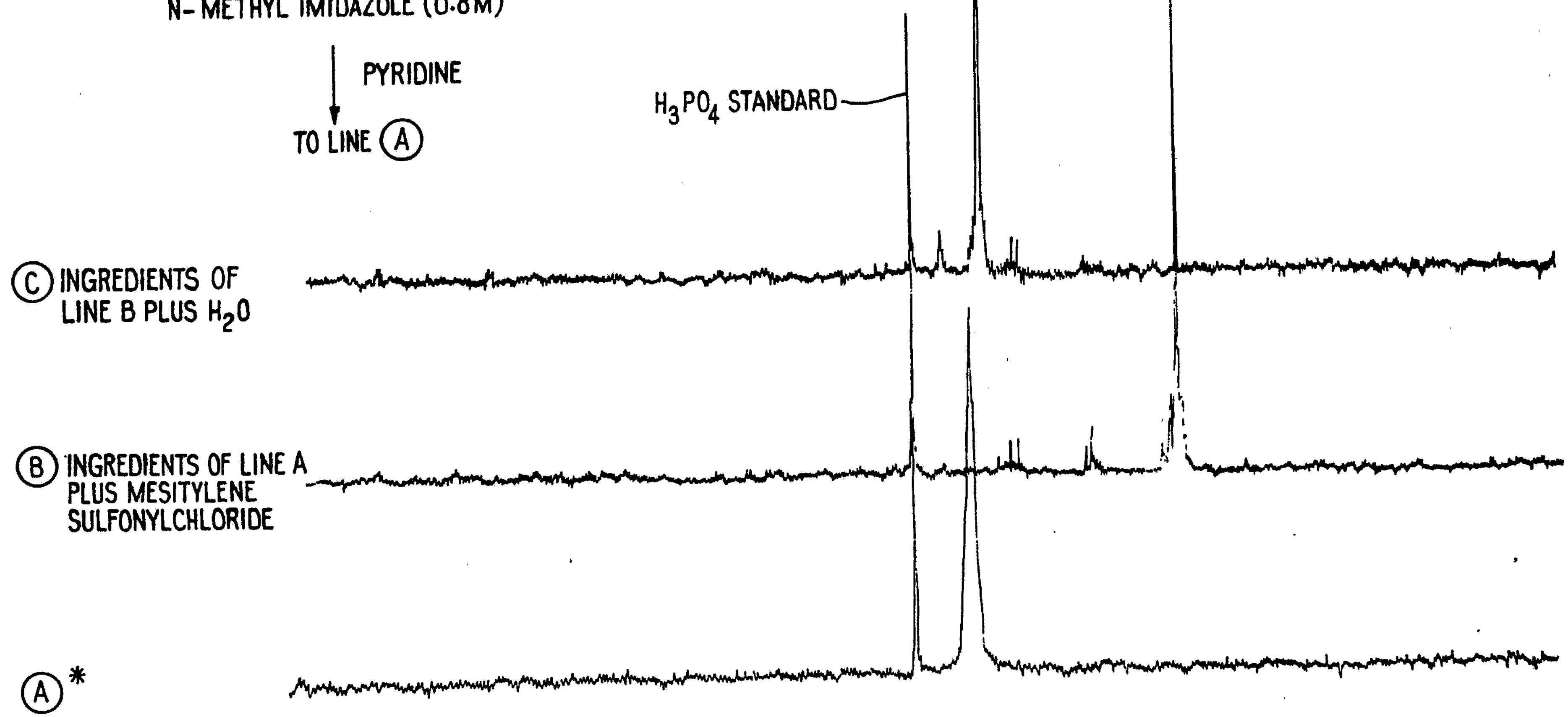

FIG.8

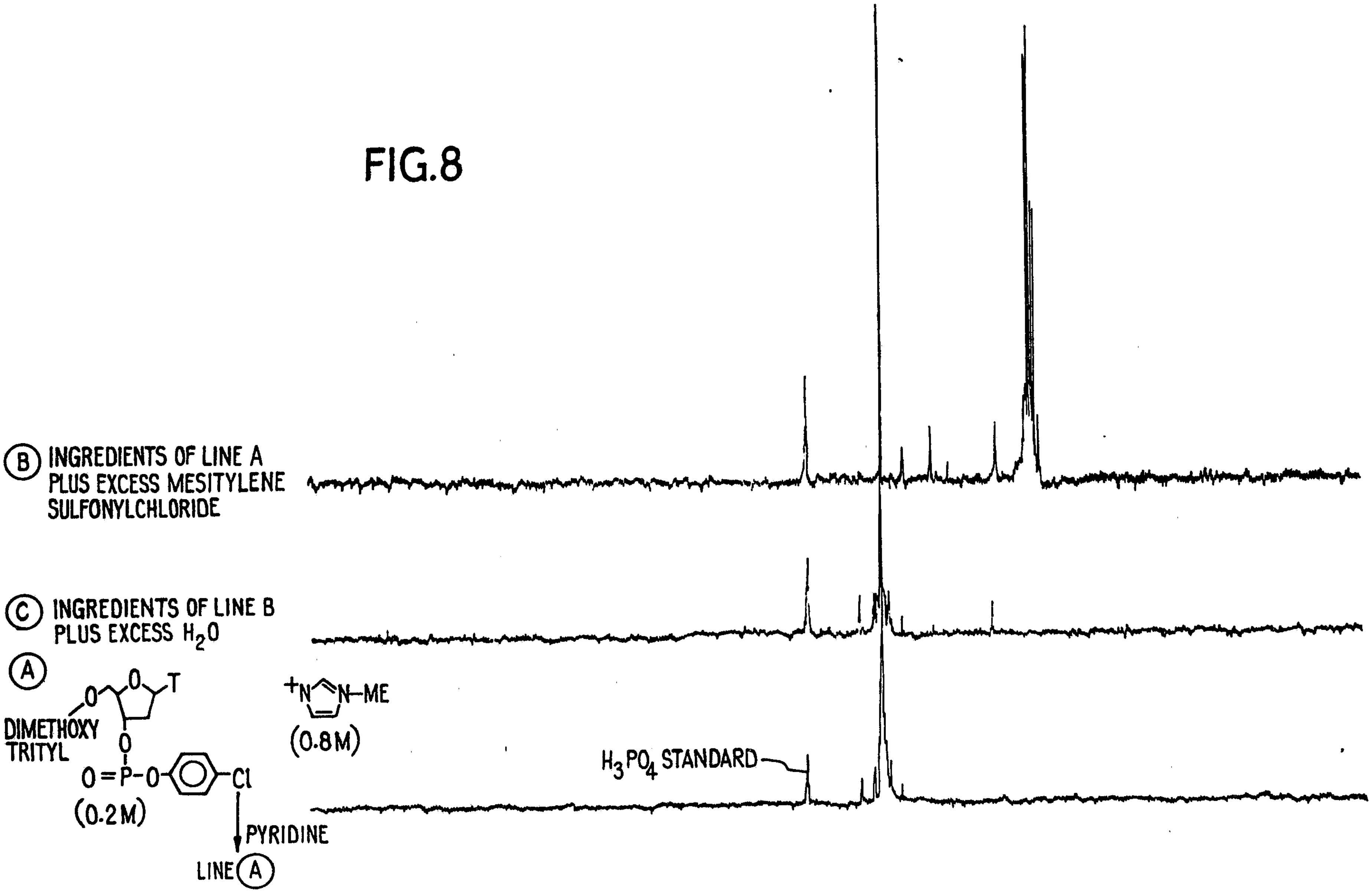
(B) INGREDIENTS OF LINE A
PLUS EXCESS MESITYLENE
SULFONYLCHLORIDE
(C) INGREDIENTS OF LINE B
PLUS EXCESS $H_2O$
(A)
(0.8M)
(0.2M)
PYRIDINE
LINE (A)
$H_3PO_4$ STANDARD

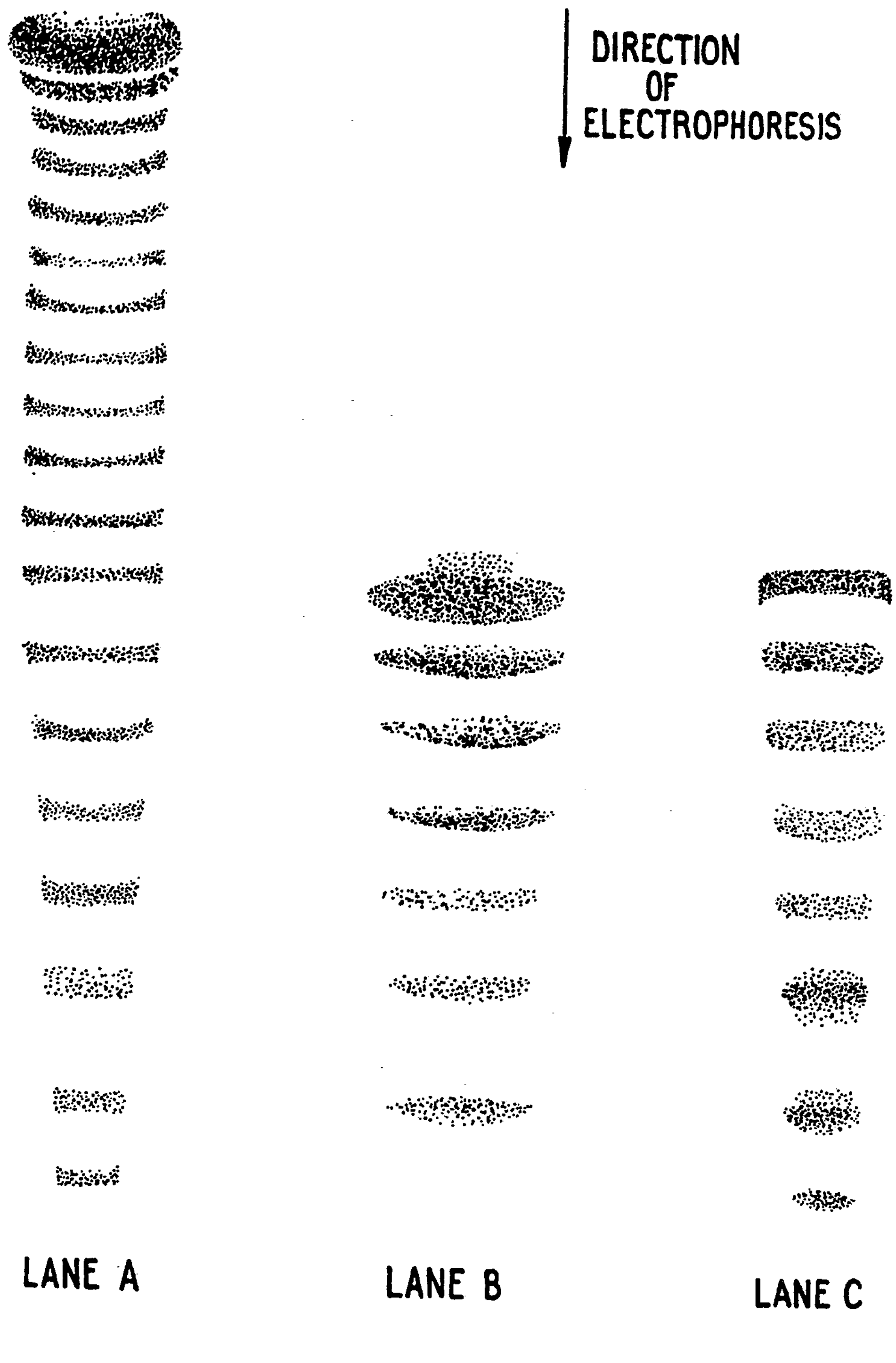


FIG.9